# EUROPEAN PATENT SPECIFICATION

(11) **EP 3 842 082 B1**
(45) Date of publication and mention of the grant of the patent: **16.11.2022**
(21) Application number: 19869650.2
(22) Date of filing: 11.09.2019
(51) Int. Cl.: A61M 1/16

(54) **DIALYSIS SOLUTION PRODUCTION DEVICE**
VORRICHTUNG ZUR HERSTELLUNG EINER DIALYSELÖSUNG
DISPOSITIF DE PRODUCTION DE SOLUTION DE DIALYSE

(30) Priority: 01.10.2018 JP 2018186859
(43) Date of publication of application: 30.06.2021
(73) Proprietor: Nihon Trim Co., Ltd., Osaka 531-0076 (JP)
(72) Inventor: NAKANISHI, Naoki, Osaka-shi, Osaka 531-0076 (JP)
(74) Representative: Germain Maureau
(86) International application number: PCT/JP2019/035773
(87) International publication number: WO 2020/071075

(56) References cited:
- EP-A1- 1 982 736
- JP-A- 2009 125 654
- JP-A- 2009 125 654
- JP-A- 2010 063 629
- JP-A- 2014 161 605
- JP-A- 2014 161 605
- JP-A- 2015 003 313
- JP-A- 2015 139 475
- JP-A- 2015 139 475
- US-A1- 2011 111 048

## Description

### TECHNICAL FIELD

The present invention relates to an apparatus for manufacturing dialysate.

### BACKGROUND ART

Hemodialysis has been known as one of the effective treatments for patients with renal failure who have decreased renal function and are unable to excrete urine to regulate water content and remove harmful substances inside the body including waste products such as urea.

Such hemodialysis is a treatment method in which the following operation is repeatedly performed: Blood is drawn out of a body using a blood pump, and the dialysate and the blood are brought into contact with each other via a dialyzer, whereby a diffusion phenomenon due to a concentration gradient is utilized to remove harmful substances in the body and water from the blood, and then the blood is returned to the body again (retransfusion).

Further, in recent years, it has been observed that oxidative stress occurs in dialysis patients in the hemodialysis. It is considered that the oxidative stress is caused by active oxygen generated during dialysis, and it has been proposed to eliminate this active oxygen to reduce the oxidative stress.

For example, an apparatus for manufacturing dialysate is disclosed according to which dialysate is prepared by dissolving hydrogen gas in dialysate source reagent (agent A containing glucose, sodium, and the like) and then mixing the dialysate source reagent with another dialysate source reagent (agent B containing bicarbonate). It is disclosed that such configuration makes it possible to manufacture dialysate with lowered oxidation-reduction potential (see, for example, Patent Document 1).

### CITATION LIST

### PATENT DOCUMENTS

PATENT DOCUMENT 1: Japanese Patent No. 5872321

EP 1982 736 A1 relates to a dialysis equipment including means for supplying dialysis solution preparation water having a dissolved hydrogen concentration of 50 to 600 ppb, a pH of 7-10, and satisfying the water quality criterion defined at ISO 13959, means for storing a dialysis base agent including at least 50 ng/mL of a glucose degradation product, and means for preparing the dialysis solution by diluting the dialysis base agent with said dialysis solution preparation water.

JP 2010 063629 A relates to a dialyzer comprising a gas/liquid mixing means for mixing hydrogen in water and pressurizing the mixture to dissolve hydrogen in water; a microbubble generating means for generating microbubbles under water acquired by the gas/liquid mixing means in which hydrogen is dissolved; and a dialysate feeding means for feeding water including microbubbles acquired by the microbubble generating means as the dialysate.

JP 2015 139475 A relates to an apparatus for manufacturing water for dialysis fluid preparation including: a tank for storing water; an electrolytic water generator connected to the tank , which is a hydrogen dissolution device for dissolving hydrogen in the water introduced from the tank ; a reverse osmosis membrane processor connected to the electrolytic water generator for subjecting the water in which hydrogen is dissolved to reverse osmosis membrane treatment; and a circulation passage for circulating reverse osmosis water treated by the reverse osmosis membrane processor and introducing it into the tank. The electrolytic water generator dissolves hydrogen in the water containing the reverse osmosis water introduced from the tank.

JP 2009 125654 A relates to a method comprising dissolving hydrogen into raw water by feeding the raw water to a raw water streaming part of a hydrogen gas dissolving module which is partitioned into the raw water streaming part and a hydrogen gas streaming part with a gas-permeable membrane composed of a hydrophobic material while feeding pressurized hydrogen gas to the hydrogen gas streaming part of the hydrogen gas dissolving module, thereafter filling the raw water dissolved with hydrogen gas ejected from the raw water streaming part of the hydrogen gas dissolving module into a container, sealing it, and sterilizing it.

JP 2014 161605 A relates to a hydrogen addition device including: a flow passage formed of a gas permeable membrane which selectively allows hydrogen to permeate for delivering a liquid; and a hydrogen addition part for enclosing a part of the flow passage with a hydrogen transporting fluid, and adding hydrogen to the liquid by making the hydrogen contained in the hydrogen transporting fluid permeate through the gas permeable membrane.

### SUMMARY OF THE INVENTION

### TECHNICAL PROBLEM

However, in the apparatus for manufacturing according to Patent Document 1, there has been known the following problem: In preparing the dialysate, the dialysate source reagent containing hydrogen gas is mixed with the other dialysate source reagent and then diffused, resulting in that the concentration of dissolved hydrogen in acral dialysate (i.e., dialysate supplied to a dialysis device and used for purification of patient's blood through dialyzer) decreases.

Therefore, the present invention has been made in view of the above problems, and an object of the present invention is to provide an apparatus for manufacturing dialysate, which can effectively reduce a decrease in the concentration of dissolved hydrogen in acral dialysate.

### SOLUTION TO THE PROBLEM

In order to achieve the above objective, the apparatus for manufacturing dialysate of the present invention includes: a reverse osmosis membrane treatment device configured to perform reverse osmosis membrane treatment to water; a dialysate supply device connected to the reverse osmosis membrane treatment device, and configured to prepare and supply dialysate obtained through mixing reverse osmosis water treated by the reverse osmosis membrane treatment device with undiluted dialysate; and a dialysis device connected to the dialysate supply device and configured to be supplied with the dialysate from the dialysate supply device, in which a hydrogen dissolution device configured to dissolve hydrogen gas in the dialysate and is interposed between the dialysate supply device and the dialysis device, and the hydrogen dissolution device is connected to the dialysate supply device and the dialysis device, and includes: an air supply module configured to dissolve hydrogen gas in the dialysate; and a hydrogen supply device connected to the air supply module and configured to supply hydrogen gas to the air supply module.

### ADVANTAGES OF THE INVENTION

According to the present invention, a decrease in the concentration of dissolved hydrogen in acral dialysate can be effectively reduced.

### BRIEF DESCRIPTION OF THE DRAWINGS

FIG. 1 is a schematic view showing a configuration of an apparatus for manufacturing dialysate according to a first embodiment of the present invention.
FIG. 2 is a schematic view showing a configuration of an apparatus for manufacturing dialysate according to a second embodiment of the present invention.
FIG. 3 is a flowchart for explaining a method of adjusting the concentration of dissolved hydrogen by the hydrogen dissolution device according to the second embodiment of the present invention.
FIG. 4 is a schematic diagram of a configuration of an apparatus for manufacturing dialysate according to a variation of the present invention.

### DESCRIPTION OF EMBODIMENTS

### (First Embodiment)

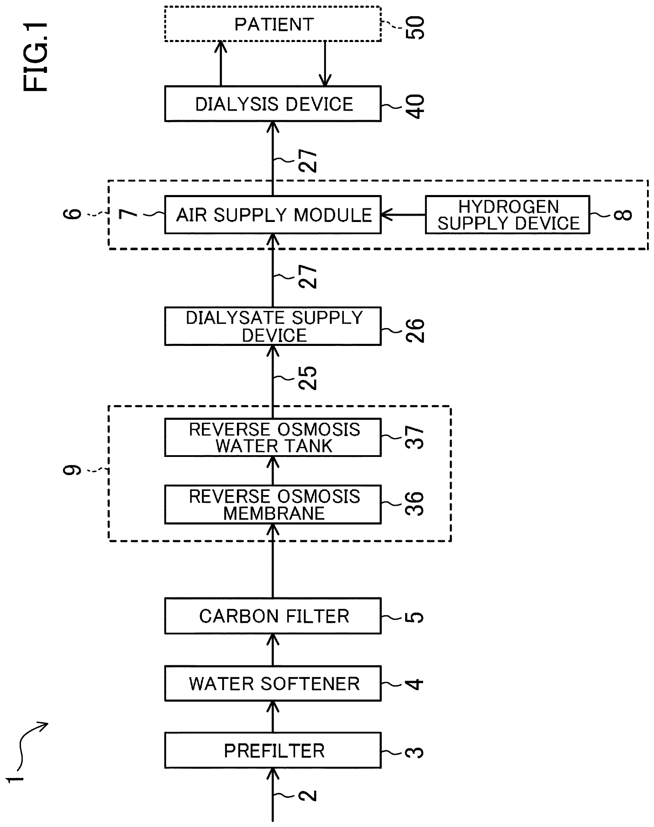

FIG. 1 is a schematic view showing a configuration of an apparatus for manufacturing dialysate according to a first embodiment of the present invention.

The apparatus 1 for manufacturing dialysate includes a prefilter 3, a water softener 4 connected to the prefilter 3, a carbon filter (active carbon treatment device) 5 connected to the water softener 4, a reverse osmosis membrane treatment device 9 connected to the carbon filter 5, and a dialysate supply device 26 connected to the reverse osmosis membrane treatment device 9.

The prefilter 3 removes impurities (for example, iron rust, sand particles, etc.) from raw water 2 (hard water containing dissolved solids such as calcium ions and magnesium ions as hardness components).

The water softener 4 performs demineralization treatment by removing hardness components from the raw water 2 through substitutional reaction due to ion exchange. Note that the raw water 2 in the present embodiment may be tap water, well water, or groundwater.

The carbon filter 5 performs treatment for removing residual chlorine, chloramine, organic substances, and the like contained in the raw water that has been treated by the water softener 4 through physical adsorption action using active carbon which is a porous adsorption material.

As the water softener 4 and the carbon filter 5, known devices may be used.

The reverse osmosis membrane treatment device 9 performs the following treatment (reverse osmosis treatment): When solutions with different concentrations coexist with a semipermeable membrane interposed therebetween, against a phenomenon in which water moves from a solution with lower concentration to a solution with higher concentration (i.e., osmosis), pressure is applied to the solution with higher concentration so that water move from the solution with higher concentration to the solution with lower concentration, thereby obtaining water osmosed into the solution with lower concentration.

Since impurities such as trace metals can be further removed from the raw water obtained by the above series of treatments using the reverse osmosis membrane treatment device 9, it is possible to obtain water (reverse osmosis water) that satisfies the water quality standard specified in ISO13959 (water standard for dialysis).

As shown in FIG. 1, the reverse osmosis membrane treatment device 9 includes a reverse osmosis membrane 36 that performs the above reverse osmosis membrane treatment to the raw water that has been subjected to a treatment performed by the carbon filter 5, and a reverse osmosis water tank 37 for storing the reverse osmosis water that has been subjected to the reverse osmosis membrane treatment.

Further, as shown in FIG. 1, a dialysate supply device 26 is connected to the reverse osmosis membrane treatment device 9. The reverse osmosis water 25 that has been subjected to the treatment performed by the reverse osmosis membrane treatment device 9 is supplied to the dialysate supply device 26.

In the dialysate supply device 26, the dialysate 27 is prepared by mixing the reverse osmosis water 25 supplied and undiluted dialysate. The dialysate 27 thus obtained is supplied to the dialysis device 40 connected to the dialysate supply device 26 to purify the blood of the patient 50. That is, the dialysate supply device 26 also functions as a device that supplies the dialysate 27 prepared to the dialysis device 40.

The undiluted dialysate may be used alone, or two or more kinds of undiluted dialysate may be used in combination.

The dialysis device 40 includes a dialyzer (not shown) that is a blood purification device for purifying blood. When hemodialysis is performed, the blood of the patient 50 is first sent to the dialyzer. Then, the purification of blood is performed by the dialyzer. Thereafter, the blood that has been purified by the dialyzer returns to the body of the patient 50.

As described above, there has been known a problem in conventional apparatuses for manufacturing dialysate that the concentration of dissolved hydrogen in acral dialysate (dialysate supplied to the dialysis device and for purifying the blood of the patient through the dialyzer) decreases.

In view of this, in the present embodiment, the dialysate supply device 26 is configured such that hydrogen gas is dissolved in the dialysate 27 that has been prepared.

More specifically, as shown in FIG. 1, in the apparatus 1 for manufacturing dialysate of the present embodiment, the hydrogen dissolution device 6 for dissolving hydrogen gas in the dialysate 27 is interposed between the dialysate supply device 26 and the dialysis device 40.

The hydrogen dissolution device 6 includes an air supply module 7 that is connected to the dialysate supply device 26 and the dialysis device 40 and dissolves hydrogen gas in the dialysate 27, and a hydrogen supply device 8 that is connected to the air supply module 7 and supplies hydrogen gas to the air supply module 7.

The air supply module 7 includes a plurality of hollow fibers connected to the dialysate supply device 26 and the dialysis device 40. The dialysate 27 that has been prepared by the dialysate supply device 26 is supplied into the inside of the hollow fibers. Further, the hydrogen supply device 8 supplies hydrogen gas to the outside of the hollow fibers. Numerous minute pores are formed in the hollow fibers such that the pores pass through the outer and inner circumferences of the hollow fibers. The walls of the hollow fibers are made of semipermeable membranes. Hydrogen gas permeates into the dialysate 27 through these semipermeable membranes. In the present embodiment, with such a configuration, the dialysate 27 with hydrogen gas dissolved therein is manufactured.

Then, hydrogen gas is supplied to the dialysate 27 while hydrogen gas flows through the outside of the hollow fibers with dialysate 27 supplied to the inside of the hollow fibers.

In addition, by using such hollow fibers, only hydrogen gas is dissolved in the dialysate 27. Thus, it is possible to reduce a disadvantageous problem that bacteria or the like are mixed into the dialysate 27 to contaminate the dialysate 27.

The hydrogen supply device 8 is not particularly limited as long as it can supply hydrogen gas to the air supply module 7. For example, a hydrogen generator that electrolyzes water, a hydrogen generating agent such as magnesium hydride that reacts with water to generate hydrogen, a hydrogen gas cylinder, and the like can be used.

As described above, in the present embodiment, the hydrogen dissolution device 6 for dissolving hydrogen gas in the dialysate 27 is interposed between the dialysate supply device 26 and the dialysis device 40.

Hence, after preparation of the dialysate 27, it is possible to dissolve hydrogen gas in the dialysate 27. Accordingly, a decrease in the concentration of dissolved hydrogen in acral dialysate 27 can be effectively reduced.

The hydrogen dissolution device 6 only includes the air supply module 7 that dissolves hydrogen gas in the dialysate 27 and the hydrogen supply device 8 that supplies hydrogen gas to the air supply module 7. Hence, it is possible to supply hydrogen gas to the dialysate 27 with a simple configuration.

Further, the air supply module 7 with a plurality of hollow fibers is used to bring hydrogen gas into contact with the dialysate 27 to dissolve the hydrogen gas. Thus, hydrogen gas can be effectively supplied.

### (Second Embodiment)

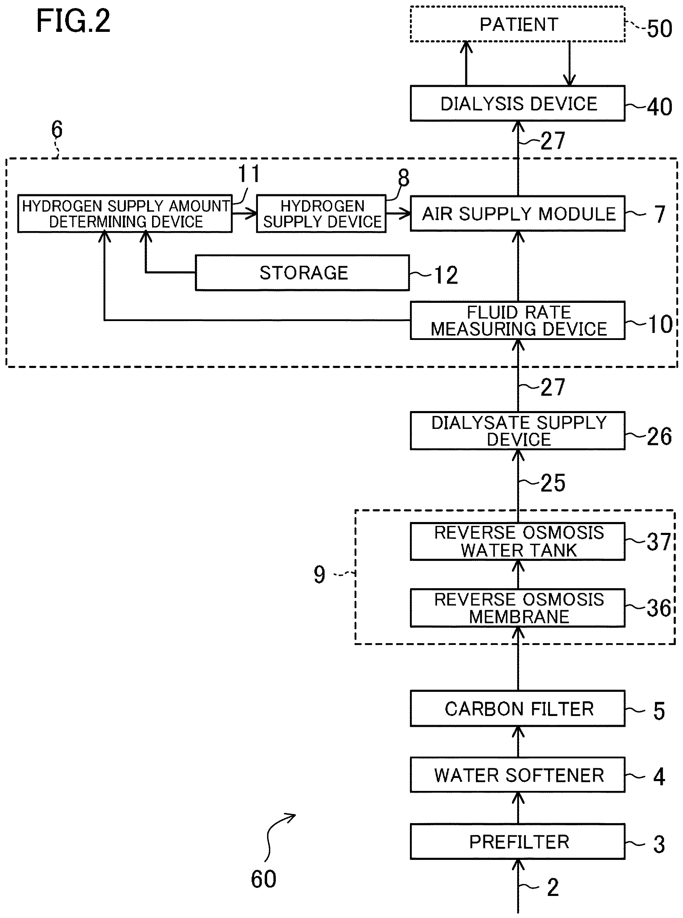

Next, a second embodiment will be described. FIG. 2 is a view showing an apparatus for manufacturing dialysate according to the second embodiment of the present invention. Note that the same reference characters as those in the first embodiment are used to represent equivalent elements, and the detailed explanation thereof will be omitted. The overall configuration of the apparatus for manufacturing dialysate is similar to that of the first embodiment described above, and thus detailed description thereof is omitted here.

In the apparatus 60 for manufacturing dialysate of the present embodiment, the hydrogen dissolution device 6 determines the amount of hydrogen to be supplied corresponding to a fluid rate (flowing fluid rate per unit time) of the dialysate 27 to be supplied to the dialysis device 40 and supplies hydrogen gas to the air supply module 7 to maintain the concentration of dissolved hydrogen in the dialysate 27 at a desired concentration.

Specifically, as shown in FIG. 2, the hydrogen dissolution device 6 according to the present embodiment includes a fluid rate measuring device 10 that is arranged on a water inflow side of the hydrogen dissolution device 6 and measures the fluid rate of the dialysate 27 supplied from the dialysate supply device 26 to the dialysis device 40, and a hydrogen supply amount determining device 11 that is connected to the fluid rate measuring device 10 and determines the amount of hydrogen supplied to the hydrogen supply device 8. Further, the hydrogen dissolution device 6 is connected to the hydrogen supply amount determining device 11 and includes a storage 12 storing data of a target value of the amount of hydrogen supply corresponding to the fluid rate of the dialysate 27.

The fluid rate measuring device 10 is not particularly limited as long as it can detect the fluid rate of the dialysate 27.

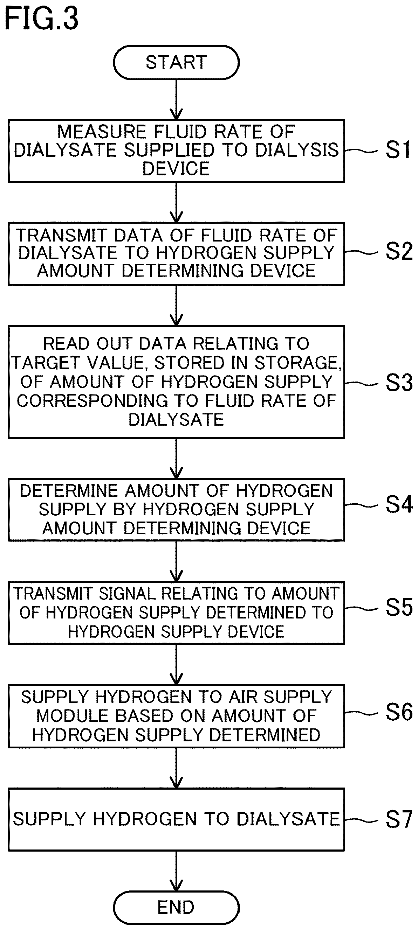

Next, a method of adjusting the concentration of dissolved hydrogen using the hydrogen dissolution device 6 will be described. FIG. 3 is a flowchart for explaining a method of adjusting the concentration of dissolved hydrogen by the hydrogen dissolution device according to the present embodiment.

First, the fluid rate measuring device 10 measures the fluid rate of the dialysate 27 supplied from the dialysate supply device 26 to the dialysis device 40 (step S1).

Then, data of the fluid rate of the dialysate 27 measured by the fluid rate measuring device 10 is transmitted to the hydrogen supply amount determining device 11 (step S2).

Next, the hydrogen supply amount determining device 11 reads out the data stored in the storage 12 on the target value of the amount of hydrogen supply corresponding to the fluid rate of the dialysate 27 (step S3).

After that, the hydrogen supply amount determining device 11 determines the hydrogen supply amount based on the value of the fluid rate of the dialysate 27 measured by the fluid rate measuring device 10 and the target value, stored in the storage 12, of the amount of hydrogen supply corresponding to the fluid rate of the dialysate 27 (step S4).

Subsequently, the hydrogen supply amount determining device 11 transmits, to the hydrogen supply device 8, a signal relating to the determined amount of hydrogen supply (step S5).

Then, the hydrogen supply device 8 supplies hydrogen to the air supply module 7 based on the hydrogen supply amount determined by the hydrogen supply amount determining device 11 (step S6). In the air supply module 7, hydrogen gas is supplied to the dialysate 27 (step S7).

As described above, according to the present embodiment, the hydrogen supply amount determining device 11 determines the amount of hydrogen supply in accordance with the value of the flow rate of the dialysate 27 measured by the fluid rate measuring device 10 and the target value, stored in the storage 12, of the amount of hydrogen supply corresponding to the flow rate of the dialysate 27. The hydrogen supply device 8 supplies hydrogen gas to the air supply module 7 based on the amount of hydrogen supply determined by the hydrogen supply amount determining device 11.

Hence, a decrease in the concentration of dissolved hydrogen in acral dialysate 27 can be effectively reduced and a desired concentration of dissolve hydrogen can be obtained.

The above embodiment may be changed as follows.

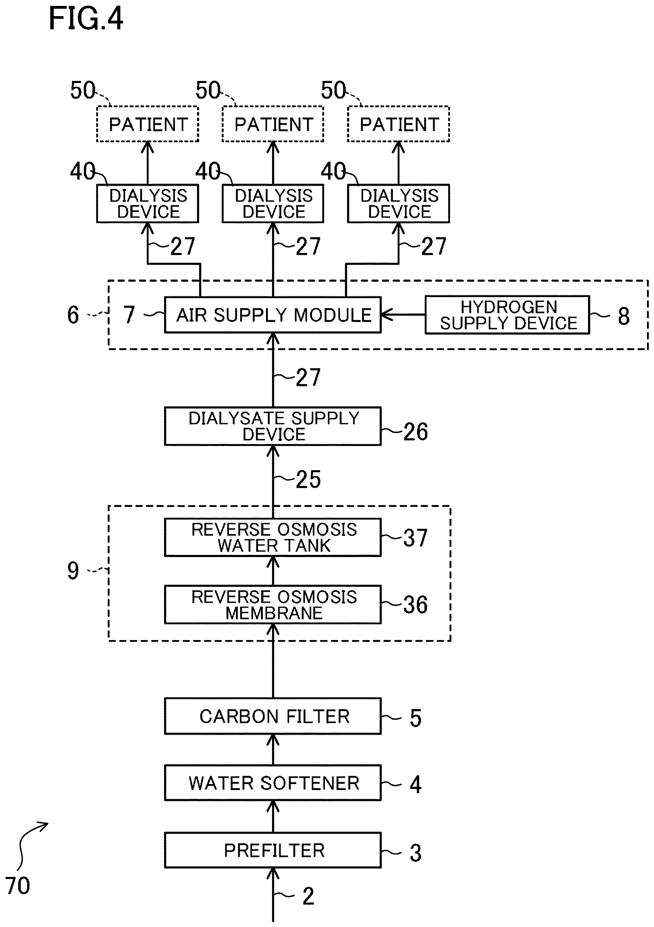

In the above embodiment, a single dialysis device 40 is connected to a single dialysate supply device 26 via the hydrogen dissolution device 6. As is the apparatus 70 for manufacturing dialysate shown in FIG. 4, a plurality of dialysis devices 40 (three in FIG. 4) may be connected to a single dialysate supply device 26 via the hydrogen dissolution device 6.

Accordingly, with a single dialysate supply device 26, it is possible to effectively reduce a decrease of the concentration of dissolved hydrogen in acral dialysate 27 (i.e., dialysate supplied to a plurality of patients 50) in the respective dialysis devices 40, and to obtain a desired concentration of dissolved hydrogen.

### INDUSTRIAL APPLICABILITY

As described above, the present invention is particularly useful for an apparatus for manufacturing dialysate with hydrogen gas dissolved therein.

### DESCRIPTION OF REFERENCE CHARACTERS

- 1: Apparatus for Manufacturing Dialysate
- 2: Raw Water
- 3: Prefilter
- 4: Water Softener
- 5: Carbon Filter
- 6: Hydrogen Dissolution Device
- 7: Air Supply Module
- 8: Hydrogen Supply Device
- 9: Reverse Osmosis Membrane Treatment Device
- 10: Fluid Rate Measuring Device
- 11: Hydrogen Supply Amount Determining Device
- 12: Storage
- 26: Dialysate Supply Device
- 27: Dialysate
- 36: Reverse Osmosis Membrane
- 37: Reverse Osmosis Water Tank
- 40: Dialysis Device
- 50: Patient
- 60: Apparatus for Manufacturing Dialysate
- 70: Apparatus for Manufacturing Dialysate

## Claims

1. An apparatus (1) for manufacturing dialysate, comprising:
a reverse osmosis membrane treatment device (9) configured to perform reverse osmosis membrane treatment to water;
a dialysate supply device (26) connected to the reverse osmosis membrane treatment device (9), and configured to prepare and supply dialysate (27) obtained through mixing reverse osmosis water treated by the reverse osmosis membrane treatment device (9) with undiluted dialysate; and
a dialysis device (40) connected to the dialysate supply device (26) and configured to be supplied with the dialysate (27) from the dialysate supply device (26),
wherein
a hydrogen dissolution device (6) configured to dissolve hydrogen gas in the dialysate is interposed between the dialysate supply device (26) and the dialysis device (40), and
the hydrogen dissolution device (6) includes an air supply module (7) that is connected to the dialysate supply device (26) and the dialysis device (40) and configured to dissolve hydrogen gas in the dialysate (27); and a hydrogen supply device (8) connected to the air supply module (7) and configured to supply hydrogen gas to the air supply module (7).

2. The apparatus (1) of claim 1, wherein the air supply module (7) includes a plurality of hollow fibers connected to the dialysate supply device (26) and the dialysis device (40), and the hydrogen gas is supplied to the dialysate (27) while the hydrogen gas flows through an outside of hollow fibers with the dialysate (27) supplied to an inside of the hollow fibers.

3. The apparatus (1) of claim 1 or 2, wherein the hydrogen dissolution device (6) includes:
a fluid rate measuring device (10) configured to measure a fluid rate of the dialysate (27) supplied to the dialysis device (40);
a hydrogen supply amount determining device (11) connected to the fluid rate measuring device (10) and the hydrogen supply device (8) and configured to determine an amount of hydrogen supply by the hydrogen supply device (8); and
a storage (12) connected to the hydrogen supply amount determining device (11) and configured to store data on a target value of the hydrogen supply amount corresponding to the fluid rate of the dialysate,
wherein
the hydrogen supply amount determining device (11) is configured to determine the amount of hydrogen supply in accordance with a value of the fluid rate of the dialysate (27) measured by the fluid rate measuring device (10) and the target value, stored in the storage (12), of the amount of hydrogen supply corresponding to the fluid rate of the dialysate (27), and
the hydrogen supply device (8) is configured to supply the hydrogen gas to the air supply module (7) based on the amount of hydrogen supply determined by the hydrogen supply amount determining device (11).

4. The apparatus (1) of claim 3, wherein the fluid rate measuring device (10) is a flow rate sensor.

5. The apparatus (1) of any one of claims 1 to 4, wherein the dialysis device (40) includes a plurality of dialysis devices.

## Patentansprüche

1. Einrichtung (1) zum Herstellen von Dialysat, umfassend:
eine Umkehrosmosemembran-Behandlungsvorrichtung (9), die dazu ausgelegt ist, Umkehrosmosemembran-Behandlung an Wasser durchzuführen;
eine Dialysat-Zufuhrvorrichtung (26), die mit der Umkehrosmosemembran-Behandlungsvorrichtung (9) verbunden und dazu ausgelegt ist, Dialysat (27), welches durch Mischen von durch die Umkehrosmosemembran-Behandlungsvorrichtung (9) behandeltem Umkehrosmosewasser mit unverdünntem Dialysat erhalten wird, zuzubereiten und zuzuführen; und
eine Dialysevorrichtung (40), die mit der Dialysat-Zufuhrvorrichtung (26) verbunden und dazu ausgelegt ist, mit dem Dialysat (27) von der Dialysat-Zufuhrvorrichtung (26) versorgt zu werden,
wobei
zwischen der Dialysat-Zufuhrvorrichtung (26) und der Dialysevorrichtung (40) eine Wasserstoffauflösevorrichtung (6) eingefügt ist, die dazu ausgelegt ist, Wasserstoffgas im Dialysat aufzulösen, und
die Wasserstoffauflösevorrichtung (6) ein Luftzufuhrmodul (7) einschließt, das mit der Dialysat-Zufuhrvorrichtung (26) und der Dialysevorrichtung (40) verbunden und dazu ausgelegt ist, Wasserstoffgas im Dialysat (27) aufzulösen; und eine Wasserstoff-Zufuhrvorrichtung (8), die mit dem Luftzufuhrmodul (7) verbunden und dazu ausgelegt ist, dem Luftzufuhrmodul (7) Wasserstoffgas zuzuführen.

2. Einrichtung (1) nach Anspruch 1, wobei das Luftzufuhrmodul (7) eine Vielzahl von Hohlfasern einschließt, die mit der Dialysat-Zufuhrvorrichtung (26) und der Dialysevorrichtung (40) verbunden sind, und das Wasserstoffgas dem Dialysat (27) zugeführt wird, während das Wasserstoffgas durch eine Außenseite von Hohlfasern strömt, wobei das Dialysat (27) einer Innenseite der Hohlfasern zugeführt wird.

3. Einrichtung (1) nach Anspruch 1 oder 2, wobei die Wasserstoffauflösevorrichtung (6) einschließt:
eine Fluidraten-Messvorrichtung (10), die dazu ausgelegt ist, eine Fluidrate des Dialysats (27), das der Dialysevorrichtung (40) zugeführt wird, zu messen;
eine Wasserstoffzufuhrmengen-Ermittlungsvorrichtung (11), die mit der Fluidraten-Messvorrichtung (10) und der Wasserstoff-Zufuhrvorrichtung (8) verbunden und dazu ausgelegt ist, eine Menge der Wasserstoffzufuhr durch die Wasserstoff-Zufuhrvorrichtung (8) zu ermitteln; und
einen Speicher (12), der mit der Wasserstoffzufuhrmengen-Ermittlungsvorrichtung (11) verbunden und dazu ausgelegt ist, Daten über einen Zielwert der Wasserstoffzufuhrmenge, welcher der Fluidrate des Dialysats entspricht, zu speichern,
wobei
die Wasserstoffzufuhrmengen-Ermittlungsvorrichtung (11) dazu ausgelegt ist, die Menge der Wasserstoffzufuhr gemäß einem Wert der Fluidrate des Dialysats (27), der von der Fluidraten-Messvorrichtung (10) gemessen wurde, und dem im Speicher (12) gespeicherten Zielwert der Wasserstoffzufuhrmenge, welcher der Fluidrate des Dialysats (27) entspricht, zu ermitteln, und
die Wasserstoff-Zufuhrvorrichtung (8) dazu ausgelegt ist, dem Luftzufuhrmodul (7) das Wasserstoffgas auf Basis der von der Wasserstuffzufuhrmengen-Ermittlungsvorrichtung (11) ermittelten Wasserstoffzufuhrmenge zuzuführen.

4. Einrichtung (1) nach Anspruch 3, wobei es sich bei der Fluidraten-Messvorrichtung (10) um einen Strömungsratensensor handelt.

5. Einrichtung (1) nach einem der Ansprüche 1 bis 4, wobei die Dialysevorrichtung (40) eine Vielzahl von Dialysevorrichtungen einschließt.

## Revendications

1. Appareil (1) pour fabriquer un dialysat, comprenant :
un dispositif de traitement par membrane d'osmose inverse (9) configuré pour réaliser un traitement par membrane d'osmose inverse de l'eau ;
un dispositif d'alimentation en dialysat (26) relié au dispositif de traitement par membrane d'osmose inverse (9), et configuré pour préparer et fournir un dialysat (27) obtenu en mélangeant de l'eau d'osmose inverse traitée par le dispositif de traitement par membrane d'osmose inverse (9) avec un dialysat non dilué ; et
un dispositif de dialyse (40) relié au dispositif d'alimentation en dialysat (26) et configuré pour être alimenté en dialysat (27) depuis le dispositif d'alimentation en dialysat (26),
dans lequel
un dispositif de dissolution d'hydrogène (6) configuré pour dissoudre de l'hydrogène gazeux dans le dialysat est interposé entre le dispositif d'alimentation en dialysat (26) et le dispositif de dialyse (40), et
le dispositif de dissolution d'hydrogène (6) comprend un module d'alimentation en air (7) qui est relié au dispositif d'alimentation en dialysat (26) et au dispositif de dialyse (40) et configuré pour dissoudre l'hydrogène gazeux dans le dialysat (27) ; et un dispositif d'alimentation en hydrogène (8) relié au module d'alimentation en air (7) et configuré pour fournir de l'hydrogène gazeux au module d'alimentation en air (7).

2. Appareil (1) selon la revendication 1, dans lequel le module d'alimentation en air (7) comprend une pluralité de fibres creuses reliées au dispositif d'alimentation en dialysat (26) et au dispositif de dialyse (40), et l'hydrogène gazeux est fourni au dialysat (27) tandis que l'hydrogène gazeux s'écoule à travers un extérieur des fibres creuses avec le dialysat (27) fourni à un intérieur des fibres creuses.

3. Appareil (1) selon la revendication 1 ou 2, dans lequel le dispositif de dissolution d'hydrogène (6) comprend :
un dispositif de mesure de débit de fluide (10) configuré pour mesurer un débit de fluide du dialysat (27) fourni au dispositif de dialyse (40) ;
un dispositif de détermination de quantité d'alimentation en hydrogène (11) relié au dispositif de mesure de débit de fluide (10) et au dispositif d'alimentation en hydrogène (8) et configuré pour déterminer une quantité d'alimentation en hydrogène par le dispositif d'alimentation en hydrogène (8) ; et
une mémoire (12) reliée au dispositif de détermination de quantité d'alimentation en hydrogène (11) et configurée pour stocker des données sur une valeur cible de la quantité d'alimentation en hydrogène correspondant au débit de fluide du dialysat,
dans lequel
le dispositif de détermination de quantité d'alimentation en hydrogène (11) est configuré pour déterminer la quantité d'alimentation en hydrogène conformément à une valeur du débit de fluide du dialysat (27) mesuré par le dispositif de mesure de débit de fluide (10) et à la valeur cible, stockée dans la mémoire (12), de la quantité d'alimentation en hydrogène correspondant au débit de fluide du dialysat (27), et
le dispositif d'alimentation en hydrogène (8) est configuré pour fournir de l'hydrogène gazeux au module d'alimentation en air (7) sur la base de la quantité d'alimentation en hydrogène déterminée par le dispositif de détermination de quantité d'alimentation en hydrogène (11).

4. Appareil (1) selon la revendication 3, dans lequel le dispositif de mesure de débit de fluide (10) est un capteur de débit.

5. Appareil (1) selon l'une quelconque des revendications 1 à 4, dans lequel le dispositif de dialyse (40) comprend une pluralité de dispositifs de dialyse.
